# EUROPEAN PATENT APPLICATION

(11) **EP 4 283 302 A1**
(43) Date of publication of application: **29.11.2023**
(21) Application number: 22175397.3
(22) Date of filing: 25.05.2022
(51) Int. Cl.: G01N 33/68, G01N 33/70

(54) **A METHOD FOR DETERMINING PREGNANCY OF AN ANIMAL AND TOOLS RELATED THERETO**

(71) Applicant: PetBiomics Oy, 00300 Helsinki (FI)
(72) Inventor: LOHI, Hannes, Helsinki (FI); OTTKA, Claudia, Helsinki (FI)
(74) Representative: Berggren Oy

(57) **Abstract**

The present invention relates to the fields of life sciences, medicine and physiology, more specifically breeding physiology. Specifically, the invention relates to a method for determining pregnancy of an animal and/or differentiating pseudopregnancy from pregnancy in an animal, wherein said method comprises measuring the levels of specific biomarkers in a sample obtained from an animal. Still, the present invention relates to a kit for determining pregnancy of an animal, a combination of specific biomarkers for determining pregnancy of an animal and use of at least said specific biomarkers for determining pregnancy of an animal.

## Description

### FIELD OF THE INVENTION

The present invention relates to the fields of life sciences, medicine and physiology, more specifically breeding physiology. Specifically, the invention relates to methods for determining pregnancy of an animal or differentiating pseudopregnancy from pregnancy in an animal, wherein said method comprises measuring the level of at least one specific biomarker in a sample obtained from an animal. Still, the present invention relates to a kit for determining pregnancy of an animal, at least one specific biomarker for determining pregnancy of an animal and use of at least one of said specific biomarkers for determining pregnancy of an animal.

### BACKGROUND OF THE INVENTION

Pregnancy diagnostics can be challenging due to the mild symptoms of early pregnancy. These symptoms can go unnoticed or similar symptoms caused by other factors may be misinterpreted as pregnancy. Reliable pregnancy detection is important, since it enables undertaking lifestyle measures to ensure reproductive safety, reproductive success and the health and normal development of the fetus(es).

Canine pregnancy is especially difficult to detect, since dogs have a state of pseudopregnancy following oestrus. The canine corpus luteum persists after oestrus, causing a similar hormonal environment than canine pregnancy. In addition to having a similar hormonal environment as pregnancy, the symptoms of overt pseudopregnancy mimic those of pregnancy, including weight gain, decreased appetite, vomiting, changes in physical activity and nesting behaviour. Thus, accurate pregnancy diagnostics are extremely important in dogs.

Several methods for diagnosing canine pregnancy are available in the art. Diagnostic methods range from palpation to imaging techniques including ultrasound and radiography, and serology. Different diagnostic tests have their own use cases. Ultrasound and serological tests are typically used for the confirmation of pregnancy during early pregnancy, and radiography for the determination of fetal number during late pregnancy. Of these methods, serological testing is the only method able to be reliably used out-of-clinic or in veterinary practice with minimal equipment and offers the possibility for consumer testing. Serological tests can be based on certain metabolites, certain hormones, or certain acute-phase proteins. However, there remains a significant need for methods and tools for determining pregnancy of animals. Indeed, effective, sensitive, and specific methods for detecting pregnancy are needed.

### BRIEF DESCRIPTION OF THE INVENTION

The objects of the invention are achieved by utilizing very specific markers for determining pregnancy, of an animal. In addition, the objects of the invention are achieved by utilizing very specific markers for differentiating pseudopregnancy from pregnancy in an animal.

It has now been found that one or more specific biomarkers can be used for determining or detecting pregnancy of an animal and/or differentiating pseudopregnancy from pregnancy in an animal. The present invention is based on the idea of measuring concentrations of one or more specific biomarkers from the sample of an animal.

The method, test or assay of the present invention is very fast, reliable, and cost effective, and can easily be carried out all over the world. The repeatability and precision of the method, test or assay of the present invention are surprisingly excellent.

A further object of the present invention is to provide modern tools and methods for both veterinarians and private people for determining pregnancy of animals and/or differentiating pseudopregnancy from pregnancy in animals.

The present invention relates to a method for determining pregnancy of an animal, which is a non-human mammal, wherein the method comprises measuring the concentration of one or more biomarkers selected from the group consisting of glycoprotein acetyls, albumin, creatinine, alanine, and glutamine, in a sample obtained from an animal, which is a non-human mammal, and determining a reproductive state of the non-human mammal using the concentration(s) of said one or more biomarkers.

The present invention also relates to a method for differentiating pseudopregnancy from pregnancy in an animal, which is a non-human mammal, wherein the method comprises measuring the concentration of one or more biomarkers selected from the group consisting of glycoprotein acetyls, albumin, creatinine, alanine, and glutamine, in a sample obtained from an animal, which is a non-human mammal, and determining a reproductive state of the non-human mammal using the concentration(s) of said one or more biomarkers.

Also, the present invention relates to an assay or concentration values of biomarkers for determining a pregnancy of an animal, which is a non-human mammal, wherein said assay or concentration values comprises reference intervals of one or more biomarkers selected from the group consisting of glycoprotein acetyls, albumin, creatinine, alanine, and glutamine, for determining pregnancy of a non-human mammal from a sample obtained from said non-human mammal.

Further, the present invention relates to an assay or concentration values of biomarkers for differentiating pseudopregnancy from pregnancy in an animal, which is a non-human mammal, wherein said assay or concentration values comprises reference intervals of one or more biomarkers selected from the group consisting of glycoprotein acetyls, albumin, creatinine, alanine, and glutamine, for differentiating pseudopregnancy from pregnancy in a non-human mammal from a sample obtained from said non-human mammal.

Still, the present invention relates to one or more biomarkers or any combination thereof for determining pregnancy of an animal, which is a non-human mammal, wherein said one or more biomarkers or any combination thereof is selected from the group consisting of glycoprotein acetyls, albumin, creatinine, alanine, and glutamine.

In addition, the present invention relates to one or more biomarkers or any combination thereof for differentiating pseudopregnancy from pregnancy in an animal, which is a non-human mammal, wherein said one or more biomarkers or any combination thereof is selected from the group consisting of glycoprotein acetyls, albumin, creatinine, alanine, and glutamine.

Still furthermore, the present invention relates to use of one or more biomarkers or any combination thereof or the assay or concentration values of the present invention for determining pregnancy of an animal, which is a non-human mammal, wherein said one or more biomarkers or any combination thereof is selected from the group consisting of glycoprotein acetyls, albumin, creatinine, alanine, and glutamine.

In addition, the present invention relates to use of one or more biomarkers or any combination thereof or the assay or concentration values of the present invention for differentiating pseudopregnancy from pregnancy in an animal, which is a non-human mammal, wherein said one or more biomarkers or any combination thereof is selected from the group consisting of glycoprotein acetyls, albumin, creatinine, alanine, and glutamine.

Other objects, details and advantages of the present invention will become apparent from the following drawing, detailed description, and examples.

The objects of the invention are achieved by methods, assays, compounds, and uses characterized by what is stated in the independent claims. The preferred embodiments of the invention are disclosed in the dependent claims.

### BRIEF DESCRIPTION OF THE DRAWINGS

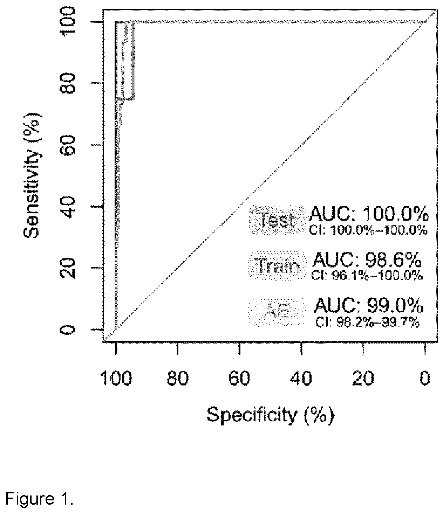

Figure 1 shows the receiver operating characteristic (ROC) curve of the multivariate model for classification of pregnant dogs. The dependent variables included in this model were albumin and glycoprotein acetyls (GlycA). An area under the curve (AUC) nearing 100% is considered excellent. Test: receiver operating characteristic (ROC) curve, AUC, and its 95% confidence intervals (CI) in testing data (pseudopregnant n = 8, pregnant n = 3), used to test the model fit in a sample set independent from the training data. Train: ROC curve, AUC, and its 95% CI in training data (pseudopregnant n = 35, pregnant n = 12), which was used to create the model. The testing and training datasets were randomly split prior to model generation from data including all pregnant (n = 15) and pseudopregnant (n = 43) dogs to cover 20% and 80% of the samples, respectively. AE: ROC curve, AUC, and its 95% CI in the data including dogs in anestrus (n = 663) and all pregnant (n = 15) dogs. This data was used to test the model in a larger dataset of female dogs.

### DETAILED DESCRIPTION OF THE INVENTION

The present invention concerns a method and a kit for determining pregnancy of an animal. The present invention also concerns a method and a kit for differentiating pseudopregnancy from pregnancy in an animal. The methods comprise measuring concentrations of one or more biomarkers from the following: glycoprotein acetyls, albumin, creatinine, alanine, and glutamine, in a sample obtained from an animal and determining pregnancy of an animal and/or differentiating pseudopregnancy from pregnancy in an animal.

As used herein "a reproductive system" refers to a system of organs working together for the purpose of sexual reproduction. In females, organs working together for the purpose of sexual reproduction include but are not limited to the organs that are directly involved in producing eggs and in conceiving and carrying babies. In males, organs working together for the purpose of sexual reproduction include but are not limited to the organs directly involved in creating, storing, and delivering sperm to fertilize an egg. Also, substances such as fluids (e.g., milk), hormones, and pheromones are important accessories to the reproductive system and are therefore within the meaning of a reproductive system.

E.g., in (domestic) dogs' sexual maturity typically occurs between the age of 6 to 18 months for both males and females, but can be delayed until up to two years of age for some large breeds. Pregnancy is possible as soon as the first estrus cycle exists.

In proestrus, the first stage of the reproductive cycle, eggs in the ovaries begin to mature and estrogen levels begin to rise. During this stage males are attracted to non-receptive females. In dogs, proestrus generally lasts 9 days. Estrus is the stage following proestrus and in estrus estrogen levels are high, mature eggs are released from ovaries, and females become ready for pregnancy. Dogs can become pregnant only during estrus. Proestrus and estrus are also known as "heat" and its length varies greatly between individuals (e.g. from 5 days to 21 days). Diestrus is the period following mating and lasts approximately 56 to 58 days in a pregnant female dog, and 60 to 100 days in a non-pregnant female. Because the hormonal profile of a pregnant female and a female in diestrus resemble each other, a non-pregnant female will sometimes go through a period of pseudopregnancy. Anestrus is the period of reproductive quiescence when a female has no attraction to or from the male. Anestrus generally lasts four to five months.

Pregnancy in a dog is 63 days in length when measured from the day of ovulation. Since the exact date of ovulation is often not tested, errors are often made in calculating pregnancy duration. As used herein "pseudopregnancy" refers to a state wherein a non-pregnant female shows symptoms of pregnancy, lactation, and/or nursing, but does not produce offspring. Due to hormonal similarities of disetrus and pregnancy, the female dog having pseudopregnancy shows these symptoms about a month or two after her heat is over.

As used herein "lactation" refers to the secretion of milk from the mammary glands in order to nurse the female's own neonates.

In the present invention pregnancy is determined by measuring or detecting biomarkers from a sample of an animal. In one embodiment of the invention the animal is a non-human mammal. In a further embodiment the mammal is selected from the group consisting of canines, felines, equids, camelids, rabbits, rodents, primates, ruminants and suids. In one embodiment, the non-human animal is a dog.

The markers used in the present invention enable determining pregnancy in an animal and/or differentiating pseudopregnancy from pregnancy in an animal.

In one embodiment the method of the present invention is an in vitro or ex vivo method. Furthermore, in one embodiment the assay of the present invention is for determining biomarkers in vitro or ex vivo.

In the present invention concentration of at least one biomarker from the following list: glycoprotein acetyls, albumin, creatinine, alanine, and glutamine, are determined from a sample. Indeed, only one biomarker or e.g., two or more biomarkers from said list can be utilized in the present invention. As used herein "glycoprotein acetyls" refer to acetyls of glycoproteins i.e., acetyl groups introduced into glycoproteins. Glycoproteins are proteins, which contain oligosaccharide chains covalently attached to amino-acid sidechains in glycosylation, which is e.g. a co-translational or post-translational modification. Examples of "glycoproteins" include α1-acid glycoprotein, haptoglobin, α1-antitrypsin, α1-antichymotrypsin, transferrin, and glycosylated apolipoproteins. Serum albumin is the most abundant blood plasma protein and is produced in the liver and forms a large proportion of all plasma protein. Serum albumins are carriers for molecules of low water solubility and furthermore they are important in regulating blood volume by maintaining the oncotic pressure. Creatinine is a chemical waste product that is mainly produced by muscle metabolism and excreted vie the kidneys. Creatinine concentration is commonly used for the estimation of renal glomerular filtration rate. Alanine and glutamine belong to the group of amino acids.

In one embodiment of the invention the methods comprise measuring the concentration of glycoprotein acetyls, albumin, creatine, alanine, or glutamine.

In another embodiment of the invention the methods comprise measuring the concentration of biomarkers
glycoprotein acetyls and albumin;
glycoprotein acetyls and creatinine;
glycoprotein acetyls and alanine;
glycoprotein acetyls and glutamine
creatinine and albumin;
creatinine and alanine;
creatinine and glutamine;
albumin and alanine;
albumin and glutamine; or
alanine and glutamine.

In a further embodiment of the invention the methods comprise measuring the concentration of biomarkers
glycoprotein acetyls, creatinine and albumin;
glycoprotein acetyls, creatinine and alanine;
glycoprotein acetyls, creatinine and glutamine;
glycoprotein acetyls, albumin and alanine;
glycoprotein acetyls, albumin and glutamine;
creatinine, albumin and alanine;
creatinine, albumin and glutamine;
creatinine, alanine and glutamine; or
albumin, alanine and glutamine.

In an even further embodiment of the invention the methods comprise measuring the concentration of biomarkers:
glycoprotein acetyls, creatinine, albumin and alanine;
glycoprotein acetyls, creatinine, albumin and glutamine;
glycoprotein acetyls, albumin; alanine and glutamine; or
creatinine, albumin, alanine and glutamine.

In one embodiment of the invention the methods comprise measuring the concentration of biomarkers:
glycoprotein acetyls, albumin, creatinine, alanine, and glutamine.

In a more specific embodiment at least said biomarker combinations are utilized for determining pregnancy or differentiating pseudopregnancy from pregnancy in an animal.

In one embodiment measuring and/or determining the concentration of one or more biomarkers reveals the exact concentration of said biomarker(s).

In one embodiment of the invention the concentration(s) of one or more biomarkers in a sample when compared to concentration(s) of the biomarker(s) in healthy non-pregnant non-human mammals is/are indicative of pregnancy of said non-human mammal. In another embodiment of the invention the concentration(s) of one or more biomarkers in a sample when compared to concentration(s) of the biomarker(s) in healthy pseudopregnant non-human mammals is/are indicative of pregnancy of said non-human mammal. In one embodiment of the invention the concentration(s) of one or more biomarkers in a sample when compared to reference interval of the biomarker(s) is/are indicative of pregnancy of said non-human mammal. In one embodiment, the healthy non-human mammal does not have an inflammatory disease.

In one embodiment of the invention the concentration(s) of the biomarker(s) in a sample are compared to a concentration of the same biomarker(s) in healthy non-pregnant non-human mammals, and an alteration in the concentration(s) of said biomarkers compared to the concentration(s) of non-pregnant non-human mammals is indicative of pregnancy. In one embodiment the alteration is an increased or decreased concentration of a biomarker or biomarkers, or a combination of an increased concentration of one or more biomarkers and a decreased concentration of one or more biomarkers when compared to the concentrations of said biomarkers in non-pregnant non-human mammals. In one embodiment, a non-pregnant non-human mammal is a pseudo pregnant non-human mammal.

In one embodiment of the invention the concentration(s) of the biomarker(s) in a sample are compared to reference interval(s) of the same biomarker(s), and an alteration in the concentration(s) of said biomarkers compared to the reference interval(s) is indicative of pregnancy. In one embodiment the alteration is an increased or decreased concentration of a biomarker or biomarkers, or a combination of an increased concentration of one or more biomarkers and a decreased concentration of one or more biomarkers when compared to the reference intervals.

In one embodiment the alteration is an increased concentration of glycoprotein acetyls when compared to non-pregnant non-human mammals or to the reference interval. In another embodiment the alteration is a decreased concentration of albumin when compared to non-pregnant non-human mammals or to the reference interval. In a further embodiment the alteration is a decreased concentration of creatinine when compared to non-pregnant non-human mammals or to the reference interval. In a further embodiment the alteration is a decreased concentration of alanine when compared to non-pregnant non-human mammals or to the reference interval. In a further embodiment the alteration is a decreased concentration of glutamine when compared to non-pregnant non-human mammals or to the reference interval. In one embodiment, a non-pregnant non-human mammal is a pseudo pregnant non-human mammal.

As used herein "reference interval" refers to concentrations of biomarkers or a typical range of concentrations determined from a set of healthy non-human mammals, regardless of the non-human mammal is male or female and regardless of reproductive state, wherein 95% of the healthy population of said non-human mammal falls within the reference interval. The biomarker values in pregnant non-human mammals might exceed or undercut these reference intervals. Indeed, a concentration of one or more biomarkers can be compared to a concentration of said one or more biomarkers, or a reference interval(s) thereof, obtained from a sample or samples of non-pregnant non-human mammals of the same species.

The concentration of the biomarker in the sample of a non-human mammal to be determined in the present invention may alter, increase or decrease compared to the concentration of the same biomarker in healthy non-pregnant non-human mammals of the same species or to the reference interval thereof. In one embodiment of the invention the alteration, increase or decrease significantly affect a statistical model based on these biomarkers. As used herein "significant" refers to statistically significant i.e. p≤ 0.05. Statistical methods suitable for the present invention are any common statistical methods known to a person skilled in the art. In a specific embodiment of the invention the statistical method for determining pseudopregnancy is logistic regression, for example Firth logistic regression.

In one embodiment, the present invention concerns an assay or concentration values of biomarkers for determining a reproductive state of an animal, which is a non-human mammal, wherein said assay or concentration values comprises reference intervals of one or more specific biomarkers for determining a reproductive state of a mammal from a sample obtained from said mammal.

In one embodiment the method of the present invention further comprises comparing the concentration values of one or more specific biomarkers to reference intervals, which are optionally obtained from an overall population of healthy animals.

In a specific embodiment if the concentrations of one or more biomarkers obtained with the method or assay of the present invention fall outside the reference intervals, pregnancy is determined.

In one embodiment of the invention the assay or concentration values comprises reference intervals of biomarkers selected from
glycoprotein acetyls;
albumin;
creatinine;
alanine; and/or
glutamine.

In one embodiment the serum reference intervals for the biomarkers are as follows:
0.597-1.028 mmol/I for glycoprotein acetyls;
25 - 32 mmol/l for albumin;
0.032-0.103 mmol/l for creatinine;
0.216-0.597 mmol/l for alanine; and
0.640-1.015 mmol/l for glutamine.

These reference intervals have been calculated based on the concentrations of the biomarkers in the whole population. The concentration values of 95% of the healthy population fall within the reference intervals.

Measuring of concentrations of the biomarkers may be carried out by any method known to a person skilled in the art including but not limited to nuclear magnetic resonance (NMR) spectroscopy, gas chromatography, mass spectrometry (MS), liquid chromatography, immunoassay (e.g., enzyme-linked immunosorbent assay (ELISA) or fluoro immunoassay (FIA)), colorimetric analysis, enzymatic or partially enzymatic assays, electrophoresis or any combination thereof. The immunoassay can be either a competitive or non-competitive immunoassay. Competitive immunoassays include homogenous and heterogenous immunoassays.

In one embodiment the method of the present invention further comprises collecting data of the animal, including but not limited to an age, breed, (overall) health, fasting duration, onset of symptoms and/or nature of symptoms. In another embodiment the method of the present invention further comprises a physical examination of the mammalian, routine blood tests (e.g., including CRP, a complete blood count and serum biochemistry) and/or urinalysis. E.g., abdominal ultrasound, physical examination and routine blood tests can be used for ruling out pyometra.

Accordingly, the assay of the present invention may optionally comprise any further data of the animal including but not limited to an age, breed, (overall) health, fasting duration, onset of symptoms, nature of symptoms, data on a physical examination of the mammalian, results of routine blood tests (e.g. including a complete blood count) and/or results of urinalysis.

Samples used in the method or assay of the present invention may be collected and optionally treated with any suitable method known to a person skilled in the art (e.g., including collecting blood and optionally separating plasma or serum from said blood). In one embodiment of the invention the sample is a blood sample, plasma sample, serum sample, saliva sample or urine sample. In another embodiment, the sample is a blood, plasma or serum sample.

Any suitable amount of a sample can be used for the method or assay of the present invention but in one embodiment of the invention amount of a sample for the method or assay is about 50 µl - 1000 µl, e.g., about 100 µl - 500 µl or about 100 µl - 300 µl. Indeed reliable, sensitive and specific results can be obtained with a relatively small amount of a sample, which makes the present invention both user-friendly and effective.

In one embodiment of the invention the assay further comprises calculation means for determining pregnancy of the mammal by using the input concentration of said biomarkers, and optionally data input means for inputting a concentration of said one or more biomarkers in a sample of the mammal for calculation. In one embodiment calculation means includes but is not limited to means for carrying out a statistical analysis for determining pregnancy e.g., based on the concentration of the biomarker(s) in a sample and optionally a reference interval. In a specific embodiment calculation means calculates the pregnancy status of the mammal by using the concentration of said biomarkers in a sample, and optionally reference interval.

In a specific embodiment the assay or concentration values of the present invention is/are for the method of the present invention.

The tools or assay of the present invention may be produced by one or several conventional processes known in the art and may comprise any components normally found in corresponding products. In a specific embodiment the assay comprises instructions for carrying out a method for determining a reproductive state of a mammal. E.g., said instructions may include instructions selected from the group consisting of instructions for carrying out the assay, instructions for selecting the relevant biomarkers, instructions for interpreting the results i.e., concentrations and/or particle sizes of one or more biomarkers (optionally compared to normal concentrations and/or particle sizes, and/or reference values), and instructions for carrying out the statistical analysis.

The biomarkers, method or analysis of the present invention may be utilized for any mammalian e.g., without any prior physical examination or tests. In one embodiment the biomarkers, methods or analysis of the present invention are utilized for an animal which is found healthy or can be considered healthy. In one embodiment before classifying an animal as suitable for the method or assay of the present invention, a veterinarian, or any other person skilled in the art may for example study the condition or symptoms of the animal and/or assay any biomarkers (e.g., biomarkers of normal physiological conditions and/or diseases). Furthermore, based on the results obtained by using the biomarkers, method or assay of the present invention, the veterinarian or person skilled in the art may suggest e.g., follow-up, further tests, operations or actions for the animal, if needed.

It will be obvious to a person skilled in the art that, as the technology advances, the inventive concept can be implemented in various ways. The invention and its embodiments are not limited to the examples described below but may vary within the scope of the claims.

### EXAMPLES

A method for the detection of canine pregnancy was aimed at identifying the presence of pregnancy based on metabolite data. The method was based on a previously collected sample set of 6306 serum/plasma samples from privately owned pet dogs as described in more detail in (Ottka C. et al., Veterinary Clinical Pathology, n/a(50), 410-426, 2021).

A subset of this dataset, including only appropriately separated serum samples from healthy, unspayed female dogs aged 2-9 years old with known and uncon-flicting information on the reproductive state was used in demonstrating the applicability of the method. The samples were analyzed using a validated, dog-specific proton nuclear magnetic resonance (¹H NMR) spectroscopy-based metabolomics platform described in (Ottka et al. 2021) and the quantitative metabolite data derived from the ¹H NMR spectroscopy analysis were used to detect the presence of pregnancy.

In the proof-of-concept study, the data was reviewed missing observations and metabolites with over 35% missing observations and three samples that missed all lipid results were removed from the proof-of-concept study. The data was reviewed for outliers and eight severely lipemic samples were removed. The final dataset in the proof-of-concept study included samples of 663 dogs in anestrus, 78 dogs in heat, and 43 pseudopregnant, 15 pregnant, and 38 lactating dogs. The ages and fasting durations in all groups were tested to be similar using the Kruskal-Wallis test. Remaining missing metabolite values were imputed using random forest imputation. According to visual distribution checking, most of metabolites were not normally distributed.

Differences in metabolite concentrations between anestrus, heat, pseudopregnancy, pregnancy, and lactation was analyzed using the Kruskal-Wallis test with the significance threshold obtained from Bonferroni-correction based on the number of principal components explaining 95% of the variation in the data, in this case p < 0.0026 (0.05/19). Post-hoc comparisons were conducted using the Dunn test with p-values adjusted using Bonferroni-correction based on the number of individual comparisons with a significance threshold of p < 0.05. Differences in metabolite concentrations between pregnancy and pseudopregnancy were observed for Alanine, Glutamine, Albumin, Creatinine, Docosapentaenoic acid % (DPA%), and GlycA and are shown in Table 1.

**Table 1. Metabolites with significant differences in the Kruskal-Wallis test between pregnancy and pseudopregnancy. DPA: docosapentaenoic acid.**

| Metabolite | Pregnant median (range) | Pseudopregnant median (range) | Reference interval | P-value overall | Post-hoc p-value |
|---|---|---|---|---|---|
| Alanine mmol/l | 0.323 (0.180-0.381) | 0.395 (0.240 - 0.564) | 0.216-0.597 | < 0.0001 | 0.04 |
| Glutamine mmol/l | 0.713 (0.538 - 0.936) | 0.828 (0.646-1.035) | 0.640-1.015 | < 0.0001 | < 0.01 |
| Albumin g/l | 25 (22 - 28) | 29 (26 - 32) | 25-32 | < 0.0001 | < 0.01 |
| Creatinine mmol/l | 0.040 (0.021 - 0.061) | 0.068 (0.040-0.111) | 0.032-0.103 | < 0.0001 | < 0.01 |
| DPA % | 1.4 (1.1 - 1.8) | 1.7 (1.3 - 2.2) | 1.1-2.0 | < 0.0001 | < 0.01 |
| GlycA mmol/l | 1.024 (0.733 - 1.216) | 0.717 (0.573-1.100) | 0.597-1.028 | < 0.0001 | < 0.01 |

In the proof-of concept study, the ability of metabolite concentrations to differentiate pregnant dogs from pseudopregnant ones was evaluated by creating Firth logistic regression models. To create these models, the data from pregnant and pseudopregnant dogs was split to training and testing data, covering 80% and 20% of the observations, respectively. All models were fitted using the training data. First, univariate models were created for the metabolites showing significant differences between pregnancy and pseudopregnancy in the Kruskal-Wallis test. Predictive ability of the univariate models was evaluated using the area under the curve (AUC). AUC of the testing data were used to evaluate, how well the models differentiate pregnant dogs from pseudopregnant ones in samples independent from the ones used in model creation. The measurands with highest predictive ability of the inclusion to the pseudopregnant and pregnant groups were albumin, creatinine, GlycA, glutamine, and alanine, whereas DPA% was not a suitable predictor of group inclusion (Table 2).

**Table 2. Metabolite contribution and predictive ability of univariate models based on a single metabolite. The p-values indicate the contribution of the metabolite in model specification and the area under the curve (AUC) and its confidence intervals (CI) indicate the predicitive ability of the model in testing data (test) and the dataset including all pregnant dogs and dogs in anestrus (AE).**

| Metabolite | P-value | AUCtest (CI) % | AUC_{AE} (CI) % |
|---|---|---|---|
| Albumin | 7.5E-07 | 100.0 (100.0- 100.0) | 94.9 (91.2 - 98.6) |
| GlycA | 2.1E-06 | 100.0 (100.0 - 100.0) | 93.2 (85.9 - 100.0) |
| Creatinine | 8.2E-07 | 91.7 (72.1 - 100.0) | 89.0 (82.1 - 95.9) |
| Alanine | 0.0081 | 87.5 (65.2-100.0) | 74.6 (65.4 - 83.8) |
| Glutamine | 0.00072 | 83.3 (57.3 - 100.0) | 74.7 (60.8 - 88.6) |
| DPA % | 0.73 | | |

Secondly, a multivariate Firth logistic regression model was created using forward stepwise selection based on p-values, with a threshold of < 0.05. The final multivariate model was based on albumin and GlycA. Model fit, meeting of logistic regression assumptions and the effects of age and fasting duration on the model were evaluated rigorously and the model was deemed appropriate.

The predictive ability of the final multivariate model was tested with both the testing data and in a larger dataset including the dogs in anestrus and all pregnant dogs (AE data). Confusion matrixes were generated to observe, which amount of pregnant and non-pregnant dogs were correctly classified by the model. Model sensitivity, specificity, receiver operating characteristic (ROC) curves and their AUC were also evaluated to determine, how well the model differentiates pregnant dogs from non-pregnant ones. The AUC of the model was 98.6% in the training data, 100% in the testing data and 99.0% in the AE data, which is considered excellent (Figure 1).

The sensitivity and specificity of the multivariate model based on albumin and GlycA were 100% in the testing data. In the AE data, the sensitivity was 93.3% and specificity was 97.3% (Table 3).

**Table 3. Performance of the multivariate model consisting of GlycA and albumin in the testing data and AE data.**

| | Testing data | AE data |
|---|---|---|
| Accuracy (CI) | 1 (0.715 - 1) | 0.972 (0.957 - 0.983) |
| Sensitivity | 1.000 | 0.933 |
| Specificity | 1.000 | 0.973 |

## Claims

1. A method for determining pregnancy of a non-human mammal, wherein the method comprises measuring the concentration of one or more biomarkers selected from the group consisting of
glycoprotein acetyls,
albumin,
creatinine,
alanine, and
glutamine,
in a sample obtained from the non-human mammal and determining pregnancy of the non-human mammal using the concentration(s) of said one or more biomarkers.

2. A method for differentiating pseudopregnancy from pregnancy in a non-human mammal, wherein the method comprises measuring the concentration of one or more biomarkers selected from the group consisting of
glycoprotein acetyls,
albumin,
creatinine,
alanine, and
glutamine,
in a sample obtained from the non-human mammal and differentiating pregnancy from pseudopregnancy in said non-human mammal using the concentration(s) of said one or more biomarkers.

3. The method of claim 1 or claim 2, wherein the method comprises measuring the concentrations of biomarkers
glycoprotein acetyls and albumin;
glycoprotein acetyls and creatinine;
glycoprotein acetyls and alanine;
glycoprotein acetyls and glutamine
creatinine and albumin;
creatinine and alanine;
creatinine and glutamine;
albumin and alanine;
albumin and glutamine; or
alanine and glutamine.

4. The method of any preceding claim, wherein the concentrations of at least three, four or five biomarkers are measured in a sample.

5. The method of any preceding claim, wherein the concentration(s) of one or more biomarkers in a sample when compared to concentrations of the same biomarker in healthy non-pregnant non-human mammals of the same species or to the reference interval is indicative of pregnancy of said mammal.

6. The method of any preceding claim, wherein the concentration(s) of the biomarker(s) in a sample are compared to a normal concentration of the same biomarker(s), and an alteration in the concentration(s) of said biomarkers compared to the concentration of the same biomarker(s) in healthy non-pregnant non-human mammals of the same species or to the reference interval is indicative of pregnancy.

7. The method of claim 6, wherein the alteration is an increased or decreased concentration of biomarker(s), when compared to the concentration(s) of said biomarker(s) in healthy non-pregnant non-human mammals of the same species or to the reference interval(s).

8. The method of claim 6, wherein the alteration is a combination of an increased concentration of one or more biomarkers and a decreased concentration of one or more biomarkers when compared to the concentrations of said biomarkers in healthy non-pregnant non-human mammals of the same species or to the reference interval(s).

9. The method of any preceding claim, wherein said method is an in vitro or ex vivo method.

10. An assay or concentration of biomarkers for determining pregnancy of a non-human mammal or for differentiating pseudopregnancy from pregnancy in a non-human mammal, wherein said assay or concentration comprises reference intervals of one or more biomarkers selected from the group consisting of
glycoprotein acetyls,
albumin,
creatinine,
alanine,
glutamine,
for determining pregnancy of a non-human mammal from a sample obtained from said non-human mammal.

11. The method, assay or concentration of any preceding claim, wherein the method or assay further comprises calculation means for determining the pregnancy of the non-human mammal by using the input concentration of said biomarkers, and optionally data input means for inputting a concentration of said one or more biomarkers in a sample of the non-human mammal for calculation.

12. The assay or concentration of any claim 10 or 11, wherein said assay or concentration is for the method of any one of claims 1-9.

13. The method, assay or concentration values of any preceding claim, wherein the non-human mammal is selected from the group consisting of canines, felines, equids, camelids, rabbits, rodents, primates, ruminants and suids.

14. One or more biomarkers or any combination thereof for determining pregnancy of a non-human mammal or differentiating pseudopregnancy from pregnancy in a non-human mammal, wherein said one or more biomarkers or any combination thereof is selected from the group consisting of
glycoprotein acetyls,
albumin,
creatinine,
alanine, and
glutamine.

15. Use of one or more biomarkers or any combination thereof or the assay or concentration values of any one of claims 12-14 for determining pregnancy of a non-human mammal or differentiating pseudopregnancy from pregnancy in a non-human mammal, wherein said one or more biomarkers or any combination thereof is selected from the group consisting of
glycoprotein acetyls,
albumin,
creatinine,
alanine, and
glutamine.
